# DEMANDE DE BREVET EUROPEEN

(11) **EP 2 933 637 A1**
(43) Date de publication de la demande: **21.10.2015**
(21) Numéro de dépôt: 15163895.4
(22) Date de dépôt: 16.04.2015
(51) Int. Cl.: G01N 33/46, G01N 31/12

(54) **PROCEDE ET SYSTEME DE DETERMINATION D'UN TAUX D'HUMIDITE D'UNE MATIERE ORGANIQUE UTILISEE COMME COMBUSTIBLE**

(30) Priorité: 17.04.2014 FR 1453485
(71) Demandeur: Cylergie, 92800 Puteaux (FR)
(72) Inventeur: Pascual, Christophe, 69008 Lyon (FR); Provent, Anne-Sonia, 69005 Lyon (FR); Morin, Brice, 69007 Lyon (FR)
(74) Mandataire: Novagraaf Technologies

(57) **Abrégé**

Selon un procédé de détermination d'un taux d'humidité d'une matière organique utilisée comme combustible dans une chaudière comportant un conduit de fumées (4) pour évacuer des fumées de combustion de la matière organique dans la chaudière, on réalise un prélèvement de fumées dans le conduit de fumées (4), on détermine le taux d'humidité dans les fumées prélevées et on détermine le taux d'humidité de la matière organique en fonction du taux d'humidité des fumées.

## Description

### Domaine technique

La présente invention se rapporte à un procédé de détermination d'un taux d'humidité d'une matière organique utilisée comme combustible dans une chaudière. Elle concerne également un système mettant en oeuvre un tel procédé.

### Etat de la technique

Avec les techniques actuelles, il n'est pas aisé de connaître en instantané le rendement d'une chaudière brûlant de la matière organique telle que du bois sous forme fragmentée. Avec les combustibles fossiles tels que le gaz ou le fioul, la connaissance du débit de combustible est aisée à connaître à l'aide d'instruments largement répandus.

Cependant, dans le cas de la biomasse, on est confronté à plusieurs problèmes. Le premier se rapporte à la qualité de la matière organique, qui est très variable d'un approvisionnement à l'autre, et même au sein d'un même approvisionnement. La granulométrie, la densité ainsi que le taux d'humidité évoluent fortement. En conséquence, le pouvoir calorifique de la matière organique est très changeant. Même si on connaît le débit massique ou volumique de matière organique, la liaison avec une quantité de chaleur apportée n'est pas directe.

En outre, la mesure même d'un débit massique ou volumique n'est pas aisé. Dans le cas d'un dosage volumique, par exemple par des poussoirs, le taux de remplissage et la densité variables ne permettent pas de déterminer de manière suffisamment précise la quantité de matière envoyée dans la chaudière.

Or, le paramètre le plus déterminant sur le pouvoir combustible est le taux d'humidité. Il est donc important pour connaître et suivre le processus de fonctionnement de la chaudière de connaître le taux d'humidité de la matière organique utilisée comme combustible.

Le document FR 2 983 962 A1 montre une installation de mesure de l'humidité sur des plaquettes de bois utilisées comme combustible. Les plaquettes sont acheminées à travers une tuyère, et la réflexion d'ondes électromagnétiques émises par une antenne est analysée pour en déduire un taux d'humidité. La nécessité de conduire les plaquettes dans une tuyère induit des contraintes importantes d'implantation incompatibles avec des installations existantes

L'invention vise à fournir un procédé de détermination d'un taux d'humidité d'une matière organique utilisée comme combustible dans une chaudière qui soit fiable, précis et réactif.

### Description de l'invention

Avec ces objectifs en vue, l'invention a pour objet un procédé de détermination d'un taux d'humidité d'une matière organique utilisée comme combustible dans une chaudière comportant un conduit de fumées pour évacuer des fumées de combustion de la matière organique dans la chaudière, caractérisé en ce qu'on réalise un prélèvement de fumées dans le conduit de fumées, on détermine le taux d'humidité dans les fumées prélevées et on détermine le taux d'humidité de la matière organique en fonction du taux d'humidité des fumées.

Ainsi, le prélèvement de fumées et la détermination du taux d'humidité de la matière organique peuvent être réalisés en continu. En outre, le prélèvement des fumées est effectué en un seul point du conduit de fumées, limitant ainsi les contraintes d'implantation du système selon l'invention. Le prélèvement dans les fumées permet de connaître le taux d'humidité de ces fumées, de manière directe ou indirecte, comme on le verra mieux par la suite. L'humidité des fumées provient essentiellement de l'eau vaporisée lors de la combustion de la matière organique, et d'autre part de l'humidité dans l'air comburant et de la formation d'eau pendant la combustion. De ce taux d'humidité dans les fumées, il est donc possible de remonter au taux d'humidité de la matière organique. Le taux d'humidité dans les fumées peut être mesuré directement par un capteur, par exemple par la détermination d'un point de rosée. De telles mesures peuvent être effectuées en direct et fournir des résultats en temps réel.

Selon un mode de réalisation, on réalise une première mesure d'un taux d'oxygène dans les fumées prélevées et une deuxième mesure du taux d'oxygène dans les fumées prélevées au même endroit et après un assèchement desdites fumées, le taux d'humidité dans les fumées étant déterminé en fonction des mesures des taux d'oxygène. En éliminant ou réduisant l'humidité dans les fumées prélevées, on fait varier le taux d'oxygène d'une manière qu'il est possible de déterminer par calcul. Avec un asséchement imposé, et par la mesure des taux d'oxygène, il est donc possible de calculer le taux d'humidité. Par ailleurs, en analysant le même prélèvement, on évite des fluctuations qu'il pourrait y avoir avec des prélèvements distincts, en minimisant en outre le temps de parcours des gaz et donc le risque de déphasage temporel entre les analyses.

Selon une disposition avantageuse, les fumées prélevées passent par une première sonde à oxygène pour réaliser la première mesure de taux d'oxygène, par un assécheur puis par une deuxième sonde pour réaliser la deuxième mesure de taux d'oxygène. Les mêmes fumées prélevées sont analysées d'abord par la première sonde puis par la deuxième après asséchement.

Selon une caractéristique complémentaire, on aspire les fumées par une pompe placée en aval de la deuxième sonde à oxygène. Les deux sondes sont ainsi placées sensiblement dans les mêmes conditions de pression.

De manière particulière, l'assécheur fonctionne par un refroidissement à température inférieure à 5°C.

De manière complémentaire, on déduit de l'humidité de la matière organique un pouvoir calorifique estimé de ladite matière organique. En réalisant une hypothèse sur la nature de la matière organique, par exemple en considérant que c'est du bois, on peut donner une estimation en considérant un pouvoir calorifique de matière sèche, et en diminuant ce pouvoir calorifique par la chaleur latente de vaporisation de l'eau qui correspond à l'estimation de l'humidité de ladite matière organique.

Selon une autre caractéristique complémentaire, on mesure en outre un débit massique ou volumique des fumées et un taux d'oxygène dans les fumées et on en déduit, en combinaison avec le pouvoir calorifique estimé, un débit massique de matière organique et une puissance fournie par la matière organique. Le débit des fumées est lié au débit d'air comburant et au volume fumigène de la matière organique. Le taux d'oxygène permet d'évaluer un excès d'air admis comme comburant. On en déduit le volume de fumées généré et donc le débit de matière qui est brûlée.

De manière complémentaire, on mesure la puissance délivrée par la chaudière.

Alternativement, on déduit de la mesure de débit et d'une mesure de la température des fumées une puissance de perte par les fumées, on estime par ailleurs les pertes thermiques de la chaudière et on en déduit une puissance délivrée par la chaudière en déduisant de la puissance fournie par la matière organique les puissances de perte.

Selon un perfectionnement, on retire de la puissance de la chaudière en outre une perte forfaitaire. On constate que toutes les pertes ne sont pas nécessairement prises en compte par les calculs. L'ajout d'une perte forfaitaire, évaluée après des essais de calibration, permet d'ajuster au plus près les résultats des calculs à la réalité observée.

Par exemple, la perte forfaitaire est proportionnelle à la puissance délivrée par la chaudière.

A partir de l'une de ces évaluations de la puissance de la chaudière et de la puissance fournie par la matière organique, on déduit un rendement instantané de la chaudière. Cette information est particulièrement intéressante pour vérifier le bon fonctionnement de la chaudière.

Selon l'invention, le débit de combustible est ajusté en temps réel en fonction d'au moins un des paramètres suivants : rendement instantané de la chaudière, puissance de la chaudière, puissance fournie par la matière organique. En fonction de l'objectif de fourniture d'énergie, on peut régler de manière optimale et automatique le fonctionnement de la chaudière, en particulier sur l'aspect qualité des fumées. On peut notamment faire varier, pour une même puissance à fournir, les réglages de la répartition de l'air primaire sous le combustible et la proportion d'air secondaire dans la chambre de combustion en fonction de l'humidité du combustible.

La matière organique est par exemple du bois.

L'invention a aussi pour objet un système de détermination d'un taux d'humidité d'une matière organique utilisée comme combustible dans une chaudière, la chaudière comportant un conduit de fumées pour évacuer des fumées de combustion de la matière organique, le système étant caractérisé en ce qu'il comporte des moyens de prélèvement de fumées dans le conduit de fumées, et des moyens de calcul aptes à déterminer un taux d'humidité de la matière organique en fonction d'un taux d'humidité des fumées mesuré ou déduit de mesures dans les fumées prélevées, les moyens de calculs mettant en oeuvre le procédé tel que défini précédemment.

### Brève description des figures

L'invention sera mieux comprise et d'autres particularités et avantages apparaîtront à la lecture de la description qui va suivre, la description faisant référence aux dessins annexés parmi lesquels :
- la figure 1 est un schéma d'une chaudière comportant un système selon l'invention ;
- la figure 2 est un schéma de moyens de prélèvement et de mesure sur les fumées de la chaudière de la figure 1 ;
- la figure 3 est une représentation schématique du procédé mis en oeuvre dans l'installation.

### DESCRIPTION DETAILLEE

Une chaudière pour matière organique, dite également à biomasse, comporte de manière classique une chambre de combustion 1, des moyens de convoyage 2 pour amener la matière organique dans la chambre de combustion 1, des moyens de soufflage 3 pour injecter de l'air comburant dans la chambre de combustion 1 et un conduit de fumées 4 pour évacuer les fumées de combustion.

La matière organique est amenée progressivement ou par lots par les moyens de convoyage 2 dans la chambre de combustion 1. La combustion de cette matière est réalisée à l'aide d'air comburant amené par les moyens de soufflage 3. Les fumées générées sont extraites par le conduit de fumées 4 en passant par un éventuel système de traitement, non représenté, afin de diminuer le taux de polluants. Par la suite, on considérera que la matière organique est du bois.

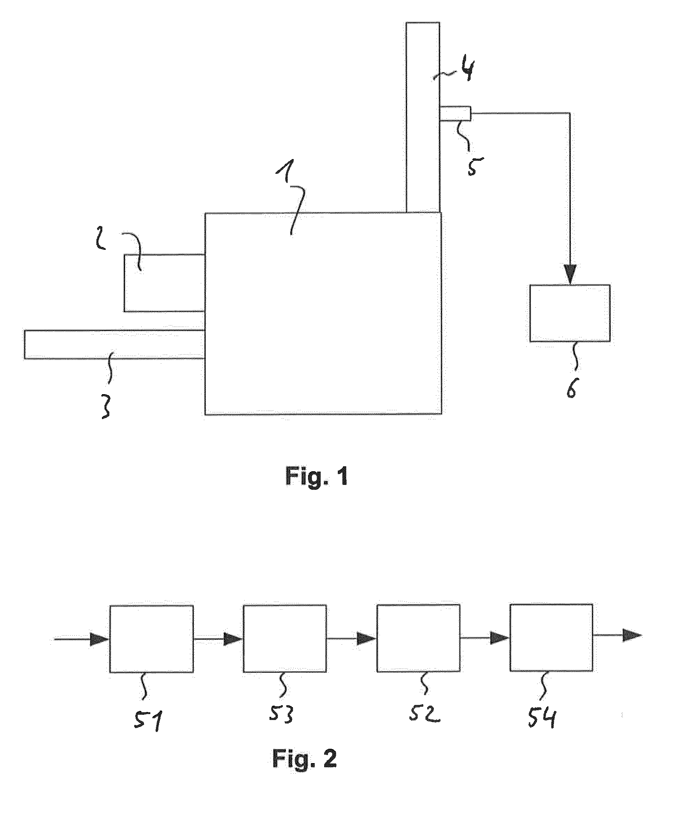

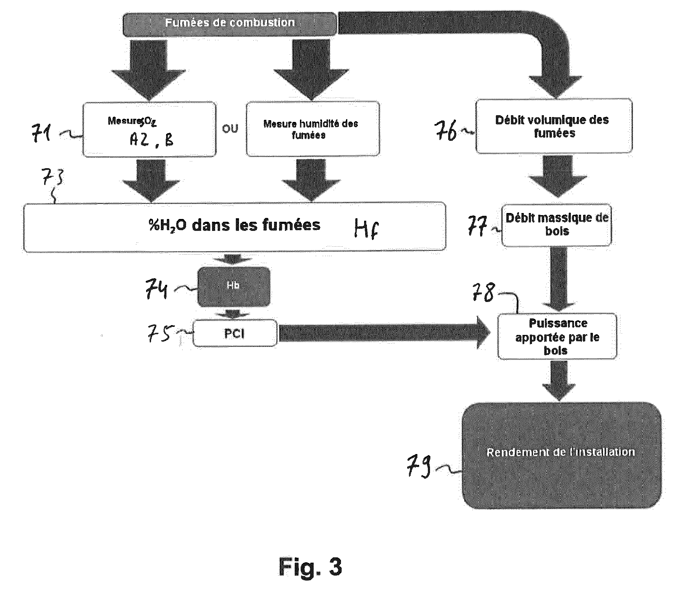

Un système de détermination de l'humidité dans la matière organique selon l'invention comporte des moyens de prélèvement 5 de fumées dans le conduit de fumées 4, et des moyens de calcul 6 aptes à déterminer un taux d'humidité de la matière organique en fonction d'un taux d'humidité des fumées déduit de mesures dans les fumées prélevées. Les moyens de prélèvement 5 des fumées sont disposés en un seul point du conduit, de préférence proche de la chaudière. Comme le montre la figure 2, les fumées prélevées passent par une première sonde 51 à oxygène pour réaliser une première mesure de taux d'oxygène B, par un assécheur 53, par une deuxième sonde 52 pour réaliser une deuxième mesure de taux d'oxygène A2, puis par une pompe 54 placée en aval de la deuxième sonde 52 à oxygène. L'assècheur 53 est par exemple un condenseur amenant les fumées à une température comprise entre 0 et 5°C. L'eau condensée est séparée et évacuée. Les mesures sont représentées par l'étape 71 du diagramme de la figure 3.

Le système selon l'invention comporte également des moyens de mesures (non représentés) de l'humidité de l'air comburant, par exemple disposés à proximité des moyens de soufflage 3.

Les moyens de calcul 6 reçoivent la première et la deuxième mesure de taux d'oxygène B, A2. Ils reçoivent en outre une mesure de la température des fumées et une mesure de débit desdites fumées. Ils reçoivent aussi la mesure de l'humidité de l'air comburant provenant des moyens de mesure de l'humidité de l'air comburant. Cette mesure permet de tenir compte de l'eau apportée par l'air comburant et donc de réaliser un calcul plus précis de l'humidité du combustible. La mesure de débit des fumées est réalisée par exemple par une sonde à ultrasons placée dans le conduit de fumées 4, non représentée.

Les moyens de calcul 6 effectuent les calculs suivants.

On exprime forfaitairement la composition du bois sec sans cendres sous la forme CₓH_{y}O_{z}NᵤSᵥ. Les valeurs usuelles des fractions massiques des différents éléments constitutifs du bois varient peu d'une essence à l'autre et on utilisera par exemple dans la suite :
**xₘ=50%** ; **yₘ=5,80%** ; **zₘ=43,20% ; uₘ=1% ; vₘ=0%**

La fraction molaire, Xᵢ de l'élément i dans le bois est calculée comme suit : ${X}_{i} = \frac{{n}_{i}}{{n}_{t}} = \frac{\frac{{fraction_massique}_{i}}{{M}_{i}}}{\sum \frac{{fraction_massique}_{i}}{{M}_{i}}}$
avec Mᵢ, masse molaire de l'élément *i*.

Au final : X=0,3271, U=0,0056, Y=0,4553, V=0 et Z=0,2120

A partir de cette composition théorique du bois, on détermine la masse de bois sec M_{bs}, en kg/mol, et les volumes comburivore sec VAS et fumigène sec VFS, c'est-à-dire le volume d'air nécessaire à une combustion stoechiométrique et le volume de fumées généré par une telle combustion, en l'absence d'eau. Ces dernières valeurs sont exprimées par exemple en Nm³/mol de bois sec.

En détail, l'équation générale de la combustion du bois est de la forme : ${C}_{x} ⁢ {H}_{y} ⁢ {O}_{z} ⁢ {N}_{u} ⁢ {S}_{v} + \left(x+v+\frac{y}{4}-\frac{z}{2}\right) ⁢ \left({O}_{2}+3,77⁢{N}_{2}\right) \to x ⁢ {CO}_{2} + \frac{y}{2} ⁢ {H}_{2} ⁢ O + v ⁢ {SO}_{2} + \left(3,77⁢\left(x+v+\frac{y}{4}-\frac{z}{2}\right)+\frac{u}{2}\right) ⁢ {N}_{2}$

En définissant un coefficient de combustion K_{c} : ${K}_{c} = x + \frac{y}{4} - \frac{z}{2}$
on détermine avec les valeurs usuelles de x, y et z :
**K_{c}=1,0225**

On obtient pour le calcul du pouvoir comburivore en air sec VAS : $VAS = 4 , 77 ⋅ {K}_{c} ⋅ {V}_{m} \left[{Nm}^{3}/mol bois sec sans cendres\right]$
avec Vₘ=0,0224 Nm³/mol le volume molaire de l'air dans les conditions normales de température et de pression (0°C et 1013 hPa). D'où :
VAS=0,1093 Nm³/mol bois sec sans cendres.

Par ailleurs, $VFS = \left(x+\frac{u}{2}+3,77⋅{K}_{c}\right) ⋅ {V}_{m} \left[{Nm}^{3}/mol bois sec sans cendres\right]$

Avec les hypothèses précédentes, on détermine :
VFS=0,1089 Nm³/mol bois sec sans cendres.

On néglige la présence des cendres car celles-ci sont relativement minimes.

Les calculs suivants sont effectués par cycles.

On peut utiliser la deuxième mesure de taux d'oxygène A2 directement comme étant le taux d'oxygène des fumées sèches A, ou le corriger en fonction de la température de refroidissement de l'assècheur 53, en considérant un gaz saturé en humidité à cette température et à une pression au niveau de l'assécheur 53, la pression étant mesurée ou prise forfaitairement.

Le pouvoir comburivore en air humide VAH est le volume d'air humide stoechiométrique nécessaire à la combustion complète d'une mole de bois.

A partir de l'humidité relative de l'air Hₐ et de la pression de vapeur saturante Pₛₐₜ à la température donnée, on peut calculer le pouvoir comburivore théorique en air humide VAH nécessaire à la combustion d'une mole de bois sec.

Pour simplifier la représentation des calculs par la suite, on adopte la notation suivante : soit PHI, la teneur en eau de l'air : $PHI = \frac{{n}_{{H}_{2} ⁢ O}}{{n}_{air sec}} \left[mol dʹeau/mol dʹair sec\right]$
or ${P}_{{H}_{2} ⁢ O} = {x}_{{H}_{2} ⁢ O} ⋅ {P}_{atm} = \frac{{n}_{{H}_{2} ⁢ O}}{{n}_{t}} ⋅ {P}_{atm} avec {P}_{H ⁢ 2 ⁢ O} la pression partielle de lʹeau \left[Pa\right]$
et ${P}_{air sec} = {x}_{air sec} ⋅ {P}_{atm} = \frac{{n}_{air sec}}{{n}_{t}} ⋅ {P}_{atm}$
d'où: $\frac{{P}_{{H}_{2} ⁢ O}}{{P}_{air sec}} = \frac{{n}_{{H}_{2} ⁢ O}}{{n}_{air sec}}$ or *P*_{*air*sec} = *Pₐₜₘ* - *P*_{*H*2*O*}
donc : $PHI = \frac{{P}_{{H}_{2} ⁢ O}}{{P}_{atm} - {P}_{{H}_{2} ⁢ O}}$ [6]

### Calcul de P_{H2O} :

${P}_{{H}_{2} ⁢ O} = \frac{{H}_{a}}{100} ⋅ {P}_{sat}$
avec : Hₐ, l'humidité relative de l'air [%] et Pₛₐₜ, la pression de vapeur saturante [Pa], obtenue par exemple par une formule empirique obtenue à partir des valeurs de tables : ${P}_{sat} = \left(0,0366.⁢{T}_{air}^{2}+0,1106.⁢{T}_{air}+6,7975\right) .101325 / 1000$

Si les valeurs de Hₐ ne sont pas accessibles en continu sur le site (pas de sonde d'humidité couplée à la mesure de T_{air ambiant}), il est possible de réaliser une approximation de P_{H2O}, par exemple par des données météo génériques du site ou d'une station proche du site.

On obtient pour le pouvoir comburivore en air humide : $VAH = VAS ⋅ \left(1+PHI\right) \left[{Nm}^{3}/mol bois sec sans cendres\right]$

A partir du taux d'oxygène sur fumées sèches, du volume comburivore sec VAS et du volume fumigène sec VFS, on détermine un excès d'air e, exprimé en pourcentage par la formule : $e = \frac{A ⋅ VFS}{VAS ⋅ \left(\frac{1}{4 , 77}-\frac{A}{100}\right)}$

La valeur 4,77 correspond au nombre de moles d'air pour une mole d'oxygène, c'est-à-dire en ajoutant 3,77 moles de diazote.

Par ailleurs, comme indiqué à l'étape 73 de la figure 3, à partir de la première mesure de taux d'oxygène B et du taux d'oxygène des fumées sèches A, on détermine un taux d'humidité des fumées *H_{f}* par la relation : ${H}_{f} = 1 - \frac{B}{A}$

A partir de l'excès d'air e, de l'humidité des fumées et de la masse molaire de bois sec, on détermine l'humidité du bois *H_{b}* lors de l'étape 74. En effet, on considère que la quantité d'eau dans les fumées provient de l'humidité contenue à l'origine dans le bois humide. L'humidité du bois est définie par la formule : ${H}_{b} = \frac{{m}_{{H}_{2} ⁢ O}}{{m}_{{H}_{2} ⁢ O} + {M}_{bs}} ⋅ 100$
avec *m_{H₂O}* la quantité d'eau en kg/mol de bois.

Si on connait l'humidité des fumées *H_{f},* on peut calculer l'humidité du bois *H_{b}* humide par les formules : ${v}_{{H}_{2} ⁢ O} = \left[\frac{{H}_{f}}{100 - {H}_{f}}.\left(VFS+\frac{e}{100}.VAS\right)-VAS.PHI.\left(1+\frac{e}{100}\right)-\frac{y}{2}.{V}_{m}\right] \left[{Nm}^{3}/mol bois\right]$
et ${H}_{b} = \frac{100}{\left(1+\frac{{M}_{bs} . {V}_{m}}{{v}_{{H}_{2} ⁢ O} . {M}_{{H}_{2} ⁢ O} ⁢ {.10}^{- 3}}\right)}$
avec *M*_{*H*₂*O*} la masse molaire de l'eau en [g/mol] ;
PHI la teneur en eau de l'air comburant, en mole d'eau par mole d'air sec ;
y la teneur en hydrogène du bois, en fraction massique ;
Vₘ le volume molaire dans les conditions normales de température et de pression, et vaut 0,0224 Nm³/mol.

On considère donc que l'eau des fumées provenant de la combustion de l'hydrogène constitutif de la matière organique n'est pas à prendre en compte dans l'humidité initiale de celle-ci. Les calculs prennent en compte l'humidité de l'air comburant, soit par des mesures directes d'humidité et de température à l'entrée des moyens de soufflage 3, soit par des données météorologiques.

A partir de l'humidité du bois, on en déduit à l'étape 75 le pouvoir calorifique inférieur du bois humide PCI. Cette donnée est essentielle pour apprécier la qualité d'un approvisionnement.

En utilisant la mesure de débit volumique des fumées, telle que figurée à l'étape 76, rapportée au pouvoir fumigène humide et à l'excès d'air, on en déduit un débit massique de bois humide à l'étape 77. En le multipliant par le pouvoir calorifique inférieur déterminé à l'étape 75, on en déduit la puissance calorifique apportée par le combustible à l'étape 78.

La puissance fournie par la chaudière *Q_{c}* peut être évaluée par exemple par la mesure d'un débit de fluide caloporteur, d'une température du fluide à l'entrée de la chaudière et une température à la sortie. En divisant cette puissance par la puissance apportée par le combustible *Q_{b},* on en déduit un rendement instantané $η = \frac{{Q}_{c}}{{Q}_{b}}$ de la chaudière à l'étape 79.

Dans un mode de réalisation des calculs, on procède ainsi : à partir de l'excès d'air, on peut calculer les volumes comburivore et fumigène réels (tenant compte de l'excès d'air) :
Volume comburivore réel en air sec VASr : ${VAS}_{r} = VAS ⋅ \left(1+\frac{e}{100}\right) \left[{Nm}^{3}/mol bois sec sans cendres\right]$
Volume fumigène sec réel VFSr : ${VFS}_{r} = VFS + VAS ⋅ \frac{e}{100} \left[{Nm}^{3}/mol bois sec sans cendres\right]$
Volume comburivore réel en air humide VAHr : ${VAH}_{r} = VAH ⋅ \left(1+\frac{e}{100}\right) \left[{Nm}^{3}/mol bois sec sans cendres\right]$
Volume fumigène humide réel VFHr (hors eau évaporée du bois) : ${VFH}_{r} = VFH + VAH ⋅ \frac{e}{100} \left[{Nm}^{3}/mol bois sec sans cendres\right]$

L'humidité du bois se définit comme le rapport de la masse d'eau qu'il contient sur sa masse brute (humide avec cendres) : ${H}_{b} = \frac{{Mʹ}_{{H}_{2} ⁢ O}}{{Mʹ}_{{H}_{2} ⁢ O} + {M}_{bs} + {Mʹ}_{c}} ⋅ 100 \left[% sur bois humide avec cendres\right]$
avec M'_{H2O} : masse d'eau par mole de bois sec sans cendres [kg/mol bois sec sans cendres], M_{bs} : masse molaire du bois sec sans cendres [kg/mol bois sec sans cendres] et M'_{c} : masse de cendres par mole de bois sec sans cendres [kg/mol bois sec sans cendres]. Cette formule, contrairement à celle exposée précédemment, prend en compte la masse de cendres. Comme déjà exposé, la masse de bois sec s'exprime : ${M}_{bs} = \left(x⁢{M}_{C}+y⁢{M}_{H}+z⁢{M}_{O}+u⁢{M}_{N}\right) \left[g/mol bois sec sans cendres\right]$
avec Mᵢ la masse molaire de l'élément i [g/mol]. Connaissant les fractions massiques des éléments x, y, z et u, on détermine : ${M}_{bs} = 24 , 028 ⁢ g / mol$

Pour calculer la masse d'eau contenue dans le bois M'_{H2O}, il faut calculer le volume d'eau évaporé du bois.

Il nous faut connaître les fractions molaires A et B, taux d'oxygène sur les fumées respectivement sèches et humides, Tₐ, VFSᵣ et VFHᵣ. Mais on peut également obtenir cette information en substituant aux valeurs de A et B celle de l'humidité des fumées Hf. Les 3 grandeurs sont liées par la relation : ${H}_{f} = \left(1-\frac{{O}_{2} ⁢ h}{{O}_{2} ⁢ s}\right) * 100 = \left(1-\frac{B}{A}\right) * 100 \left[%\right]$

Dans les paragraphes qui suivent nous présentons les formules pour les 2 méthodes.

Calcul de *Hb* avec le taux d'oxygène dans les fumées sèches et humides

On sait que la teneur en oxygène dans les fumées sèches vaut : $A = \frac{{v}_{{O}_{2}}}{{VFS}_{r}} = \frac{{O}_{2 ⁢ S}}{100} \left[%\right]$
avec *v*_{*O*₂} , volume d'O₂ dans les fumées brutes (humides)

### Correction de A :

Deux sources d'erreurs peuvent venir réduire la qualité de la mesure d'O₂sec :
- On sèche généralement les fumées à une température de 4 à 5°C, ces fumées sèches continuent donc de contenir une humidité résiduelle constante et dépendante uniquement de cette température de séchage,
- Il peut y avoir une infiltration d'air entre les 2 sondes (O₂h et O₂s) qui augmente artificiellement le taux d'oxygène sec.

On applique un coefficient de correction de la température de séchage : $corTséchage = \frac{{P}_{satH ⁢ 2 ⁢ O} \left(à Tséchage\right)}{{P}_{atm}}$
avec : Psat une pression de saturation de la vapeur d'eau dans l'air, calculée avec la formule [7a] pour Tₐᵢᵣ=T_{séchage}

Ci-dessous un tableau des valeurs de corTséchage pour une pression ambiante de 1013 hPa :

| *T_{séchage} (°C)* | *2* | *3* | *4* | *5* | *6* |
|---|---|---|---|---|---|
| *cotTséchage× 10³* | *3,05* | *3,17* | *3,33* | *3,51* | *3,73* |

Un coefficient de correction de l'infiltration est mesuré avec une bouteille de gaz étalon branchée en entrée de la sonde O₂humide, après que les 2 sondes (O₂humide et O₂sec) ont été étalonnées. $cordinfilt = \frac{{O}_{2 ⁢ Smesuré} - {O}_{2 ⁢ étalon}}{20 , 95 - {O}_{2 ⁢ Smesuré}} Coefficient de correction du débit dʹinfiltration$
avec :
- O₂S mesuré : valeur de la sonde O₂S avec le gaz étalon
- O₂ étalon : teneur en O₂ de la bouteille de gaz étalon

### O2sec corrigé :

${O}_{2 ⁢ S} ⁢ corT = \frac{{O}_{2 ⁢ S}}{\left(1-corTséchage\right)} \left[%\right] {O}_{2} ⁢ s corrigé de {T}_{séchage}$
avec : O₂s : valeur de la sonde O₂s mesurée avec la baie d'analyse. Le taux de O₂s corrigé de T_{séchage} et infiltration s'exprime : ${O}_{2 ⁢ S} ⁢ cor = {O}_{2 ⁢ S} ⁢ corT - corTinfilt * \left(20,95-{O}_{2 ⁢ S}⁢corT\right) ⁢ \left[%\right]$

Par ailleurs, la teneur en O₂ dans les fumées humides s'exprime : $B = \frac{{v}_{{O}_{2}}}{{VFH}_{r} + {v}_{{H}_{2} ⁢ O}} = \frac{{O}_{2 ⁢ H}}{100} \left[%\right]$

D'où pour v_{H2O} le volume d'eau évaporé par mol de bois : ${v}_{{H}_{2} ⁢ O} = \frac{A}{B} ⋅ {VFS}_{r} - {VFH}_{r} \left[{Nm}^{3}/mol bois sec sans cendres\right]$

Et le nombre de moles d'eau évaporées par moles de bois : ${n}_{{H}_{2} ⁢ O} = \frac{{v}_{{H}_{2} ⁢ O}}{{V}_{m}} \left[mol/mol bois sec sans cendres\right]$

On obtient alors pour m_{H20} : ${Mʹ}_{{H}_{2} ⁢ O} = {n}_{{H}_{2} ⁢ O} ⋅ {M}_{{H}_{2} ⁢ O} = \frac{{v}_{{H}_{2} ⁢ O}}{{V}_{m}} ⋅ {M}_{{H}_{2} ⁢ O} ⋅ {10}^{- 3} \left[kg dʹeau/mol bois sec sans cendres\right]$
avec *m*_{*H*₂*O*} masse molaire de l'eau en [g/mol]

Le taux cendres T_{ce} rapporté au bois sec est donné par : ${T}_{ce} = \frac{{m}_{ce}}{{m}_{bssc} + {m}_{ce}} \times 100 \left[%\right]$

Avec : m_{ce} la masse de cendres [g]
m_{bssc} la masse de bois sec sans cendre [g].

Connaissant le taux de cendres, on peut calculer la masse de cendres par mole de bois sec sans cendres : ${M}_{ce} = {M}_{bs} * \frac{{T}_{ce}}{100 - {T}_{ce}} \left[kg/mol bois sec sans cendres\right]$

Pour une mole de bois sec sans cendres, la masse M_{b} de bois brut correspondante est la somme des masses de bois sec sans cendres, de cendres et d'eau. ${M}_{b} = {M}_{bs} + {M}_{ce} + {Mʹ}_{{H}_{2} ⁢ O} \left[kg boit brut \left(humide avec cendres\right)/mol bois sec sans cendres\right]$

On en déduit l'humidité H_{b} du bois humide avec cendres, définie comme le rapport de la masse d'eau sur la masse de bois brut : ${H}_{b} = \frac{{Mʹ}_{{H}_{2} ⁢ O}}{{M}_{b}} * 100 \left[%\right]$

Dans une variante, l'humidité du bois est calculée à partir de la mesure directe de l'humidité des fumées.

Si on connaît l'humidité des fumées H_{f}, on peut calculer le volume d'eau évaporé par mole de bois sec sans cendres, par la formule [24].

Comme pour le calcul à partir des teneurs en oxygène des fumées, on en déduit la masse d'eau m_{H₂O} correspondante par l'équation [20], puis la masse de bois brut M_{b} par l'équation [22] et enfin l'humidité du bois brut H_{b} par l'équation [23].

A partir du débit de fumées humides d_{f} [Nm³/h], on peut calculer le débit molaire de bois d_{b} : ${d}_{b} = \frac{{d}_{f}}{{VFH}_{r} + {v}_{{H}_{2} ⁢ O}} \left[mol bois sec sans cendres/h\right]$

Calcul du débit massique de bois brut d_{bm} : ${d}_{bm} = {d}_{b} ⋅ {M}_{b} \left[kg bois humide avec cendres/h\right]$

Pour déterminer la puissance apportée par le combustible Qb, on utilise : $PCI = {PCI}_{anhy} ⋅ \left(1-\frac{{H}_{b}}{100}\right) - \frac{Lv ⋅ {H}_{b}}{100} \left[kJ/kg bois\right]$
avec *PCI_{anhy}*=18700 kJ/kg bois sec avec cendres : pouvoir calorifique inférieur du bois sec,
*Lv :* Chaleur latente de vaporisation=2443 kJ/kg eau (valeur à 25°C).

Puis : ${Q}_{b} = \frac{PCI ⋅ {d}_{bm}}{3600} \left[kW\right]$

Dans une variante, à partir de la mesure de débit des fumées, on évalue la puissance fournie par la matière organique en additionnant la puissance mesurée de la chaudière, celle perdue par les fumées, celle par pertes thermiques de la chaudière et en déduisant celle apportée par l'air comburant. La puissance perdue dans les fumées est évaluée à partir du débit mesuré des fumées, de leur température et d'une capacité calorifique des fumées estimée à partir des capacités théoriques de chacun des composants des fumées et de la composition des fumées. La composition est prise par exemple de manière forfaitaire pour une nature connue de bois. Elle pourrait aussi être mesurée par un analyseur de gaz pour le dioxyde de carbone, en complément de la mesure d'humidité, et éventuellement pour l'azote. Les apports par l'air comburant sont également évalués à partir de la capacité calorifique de l'air et de la température de celui-ci et de débit d'air estimé. Les pertes par rayonnement de la chaudière ne sont pas mesurées mais prises à une valeur fixe.

Dans une autre variante, lorsque la puissance de la chaudière n'est pas disponible par défaut d'instrumentation, celle-ci est estimée par l'addition de la puissance fournie par la matière organique et de celle apportée par l'air comburant, et en retirant celle perdue par les fumées et celle par pertes thermiques de la chaudière. La puissance apportée par l'air comburant, celle perdue par les fumées et les pertes thermiques sont évaluées de la même manière que précédemment. La puissance fournie par la matière organique est estimée à partir de la mesure du débit des fumées et de l'évaluation du pouvoir calorifique à partir de l'humidité, telle qu'exposée précédemment.

En détail, les calculs suivants sont effectués :

### Pertes par les fumées Q_{f}

Pour calculer les pertes par les fumées, il nous faut connaître la capacité calorifique moyenne des fumées. Pour la calculer, il nous faut connaître T_{f}, Cpᵢ, B, m_{H2O} et VFHr.

Il est nécessaire de calculer au préalable Cpᵢ, la capacité calorifique de l'élément i et xᵢ, la fraction molaire de l'élément i dans les fumées humides.

### Calcul du Cpᵢ:

${Cp}_{i} = \left[a+b⋅\frac{{T}_{f}}{1000}+c⋅{\left(\frac{{T}_{f}}{1000}\right)}^{2}+d⋅{\left(\frac{{T}_{f}}{1000}\right)}^{3}+e⋅{\left(\frac{{T}_{f}}{1000}\right)}^{- 2}\right] ⋅ {10}^{- 3} \left[kJ/mol/K\right]$
avec a, b, c, d, e des coefficients variant selon l'élément :

| | **Coefficients** | | | | |
|---|---|---|---|---|---|
| **Elément** | **a** | **b** | **c** | **d** | **e** |
| **H₂O** | 30,09 | 6,83 | 6,79 | -2,58 | 0,08 |
| **CO₂** | 25 | 55,19 | -33,7 | 7,95 | -0,14 |
| **O₂** | 29,66 | 6,14 | -1,19 | 0,1 | -0,22 |
| **N₂** | 26,09 | 8,22 | -1,98 | 0,16 | 0,04 |

### Calcul des fractions molaires :

${{x}_{{CO}_{2}}}_{fumées} = \frac{x}{{n}_{t}}$
avec ${n}_{t} = \frac{{VFH}_{r}}{{V}_{m}} + {n}_{{H}_{2} ⁢ O} \left[mol de fumées/mol bois sec sans cendres\right]$ et ${n}_{{H}_{2} ⁢ O} = \frac{{v}_{{n}_{{H}_{2} ⁢ O}}}{{V}_{m}} \left[mol dʹeau/mol bois sec sans cendres\right]$
avec v_{H2O} : volume d'eau évaporée par mol de bois sec sans cendres, calculé par les équations [19] ou [24] selon les cas. ${{x}_{{H}_{2} ⁢ O}}_{fumées} = \frac{\frac{y}{2} + 4 , 77 ⋅ PHI ⋅ {K}_{c} ⋅ \left(1+\frac{e}{100}\right) + {n}_{{H}_{2} ⁢ O}}{{n}_{t}}$

Si l'on dispose d'une mesure fiable de l'O₂h des fumées (soit parce ce qu'on réalise une mesure indirecte de l'humidité des fumées par O₂s et O₂h, soit parce qu'on dispose d'une sonde mesurant directement l'O2h) : ${x}_{{O}_{2 fumées}} = B$

Si l'on n'a pas de valeur fiable de l'O₂h, on peut la calculer à partir de l'O₂s et de humidité des fumées : ${x}_{{O}_{2 fumées}} = \frac{{O}_{2 ⁢ H}}{100} = \frac{{O}_{2 ⁢ scor}}{100} ⁢ \left(1-\frac{{H}_{f}}{100}\right)$
avec : O*_{2scor}*, O₂s corrigé (de la température de séchage de la baie utilisée, pour une mesure d'O₂s venant d'une baie classique, de la température des fumées ainsi que d'une éventuelle infiltration pour une mesure venant d'une baie O₂h/O₂s cf [17d]), et H_{f} l'humidité des fumées. Par ailleurs : ${x}_{{N}_{2 fumées}} = 1 - {x}_{{CO}_{2}} - {x}_{{H}_{2} ⁢ O} - B$

### Calcul de la capacité calorifique moyenne des fumées :

${Cp}_{f} = \left[\left(\frac{1}{{V}_{m}}\right)⋅\sum {Cp}_{i}⋅{x}_{i}\right] \left[kJ/{Nm}^{3}/K\right]$

Cette expression du Cp_{f} est mentionnée ici, mais elle n'est pas utilisée directement dans le calcul de Qf comme l'explique les lignes qui suivent.

### Calcul de Q_{f} :

${Q}_{f} = \frac{{d}_{f} . \frac{1}{{V}_{m}} ⋅ {\int }_{298}^{Tf} {Cp}_{f} ⁢ dT}{3600} \left[kW\right]$
avec d_{f} : débit de fumées humides [Nm³/h] ${d}_{f} = {d}_{b} ⁢ \left({VFH}_{r}+{v}_{{H}_{2} ⁢ O}\right) \left[{Nm}^{3} fumées humides réelles/h\right]$
T_{f} : température des fumées [K]
298 K : référence de température pour le calcul du PCI $\frac{1}{{V}_{m}} {\int }_{298}^{Tf} {Cp}_{f} ⁢ dT = \frac{1}{{V}_{m}} {\sum }_{i} {x}_{i} {\int }_{298}^{Tf} {Cp}_{i} ⁢ dT \left[kJ/{Nm}^{3}\right]$
et avec : ${\int }_{298}^{Tf} {Cp}_{i} ⁢ dT = {a}_{i} ⁢ \left({T}_{f}-298\right) + {b}_{i} ⁢ \frac{\left({T}_{f}^{2}-{298}^{3}\right)}{2000} + {c}_{i} ⁢ \frac{\left({T}_{f}^{3}-{298}^{3}\right)}{{3.10}^{6}} + {d}_{i} ⁢ \frac{\left({T}_{f}^{4}-{298}^{4}\right)}{{4.10}^{9}} - {e}_{i} ⁢ \frac{\left({T}_{f}^{- 1}-{298}^{- 1}\right)}{{10}^{- 6}} \left[kJ/mol\right]$

### Pertes par rayonnement Qᵣ

Celles-ci sont exprimées par l'équation : ${Q}_{r} = ε ⋅ S ⋅ σ ⋅ {{T}_{p}}^{4} - σ ⋅ {{T}_{a}}^{4} \left[kW\right]$
avec :
ε : émissivité des parois de la chaudière (0.8-0.9 pour de l'acier)
S : surface des parois de la chaudière [m²]
σ : constante de Stephan Boltzmann=5.67 10⁻⁸ W/m²/K⁴
Tₚ : température de paroi de la chaudière [K] (à noter que si la chaudière a plusieurs parois à des températures différentes, on ajoute les contributions pour avoir la puissance rayonnée par la chaudière)
Tₐ : température ambiante [K]

### Pertes par les cendres Qᵢ

On considère la combustion comme complète (pas d'imbrûlés dans les cendres).

Pour calculer les pertes par les cendres, il faut connaître le Cp des cendres, le débit de cendres et la température de sortie des cendres (=température de la grille). ${Cp}_{cendres} = 0 , 8 kJ / kg / K$

Le débit de cendres peut être évalué à partir du taux de cendres du bois et du débit de bois. Dans la pratique, ce terme est négligé devant les autres termes.

### Apport par l'air comburant Qₐ

Pour calculer la puissance apportée par l'air Qₐ, il faut d'abord connaître la capacité calorifique moyenne de l'air Cpₐ.

Il est nécessaire de calculer les Cpᵢ et les proportions des différents gaz dans l'air humide.

La valeur de Cpᵢ varie en fonction de la température. Dans la suite de ces calculs nous n'entrerons pas plus dans les détails, mais la température à prendre est bien la température après tous réchauffages « gratuits » (apport de chaleur provenant d'une source externe au système étudié). Sans réchauffage « gratuit », Tₐ = T_{air ambiant}, sinon Tₐ = T_{air comb} qui sera à mesurer.

### Calcul des Cpᵢ (id calcul Qf)

L'expression de la formule 31 est reprise.

### Calcul des fractions molaires :

${{x}_{{H}_{2} ⁢ O}}_{air} = \frac{{P}_{{H}_{2} ⁢ O}}{{P}_{atm}}$
avec P_{H2O} la pression de vapeur d'eau [7] ${P}_{{H}_{2} ⁢ O} = {H}_{a} * \frac{{P}_{sat}}{100} \left[Pa\right]$
ou P_{H2O} calculée à partir des données météo mensuelles, ${{x}_{{O}_{2}}}_{air} = \left(1-{x}_{{H}_{2} ⁢ O}\right) . \frac{{prop}_{{O}_{2}}}{100}$
avec prop_{O2} la proportion d'O₂ dans l'air sec = 21 % ${{x}_{{N}_{2}}}_{air} = \left(1-{x}_{{H}_{2} ⁢ O}\right) . \frac{{prop}_{{N}_{2}}}{100}$
avec prop_{N2} la proportion de N₂ dans l'air sec = 79%

### Calcul de la capacité calorifique de l'air :

${Cp}_{a} = \left[\left(\frac{1}{{V}_{m}}\right)⋅\sum {Cp}_{i}⋅{x}_{i}\right] \left[kJ/{Nm}^{3}/K\right]$
Cpₐ=1.29 kJ/Nm³/K pour de l'air à température ambiante.

Ensuite, à partir du débit d'air dₐ : ${d}_{a} = {VAH}_{r} ⋅ {d}_{b} \left[{Nm}^{3}/h\right]$
on peut calculer *Qₐ* : ${Q}_{a} = \frac{{d}_{a} . \frac{1}{{V}_{m}} ⋅ {\int }_{298}^{Ta} {Cp}_{a} ⁢ dT}{3600} \left[kW\right]$
avec Tₐ, température de l'air
298 K : référence de température pour le calcul du PCI $\frac{1}{{V}_{m}} {\int }_{298}^{Ta} {Cp}_{a} ⁢ dT = \frac{1}{{V}_{m}} {\sum }_{i} {x}_{i} {\int }_{298}^{Ta} {Cp}_{i} ⁢ dT \left[kJ/{Nm}^{3}\right]$
et avec : ${\int }_{298}^{Ta} {Cp}_{i} ⁢ dT = {a}_{i} ⁢ \left({T}_{a}-298\right) + {b}_{i} ⁢ \frac{\left({T}_{a}^{2}-{298}^{3}\right)}{2000} + {c}_{i} ⁢ \frac{\left({T}_{a}^{3}-{298}^{3}\right)}{{3.10}^{6}} + {d}_{i} ⁢ \frac{\left({T}_{a}^{4}-{298}^{4}\right)}{{4.10}^{9}} - {e}_{i} ⁢ \frac{\left({T}_{a}^{- 1}-{298}^{- 1}\right)}{{10}^{- 6}} \left[kJ/mol\right]$

### Calcul de Q_{b}

${Q}_{b} = {Q}_{f} + {Q}_{c} + {Q}_{r} + {Q}_{cd} - {Q}_{a} \left[kW\right]$
avec :
Q_{c}, la puissance fournie par la chaudière (au niveau du compteur)
Q_{cd}, une puissance forfaitaire et artificielle de pertes proportionnelles à l'allure et qui est sensée prendre en compte toutes les pertes proportionnelles dont on ne tient pas compte dans la méthode indirecte, ce Q_{cd} a pour rôle de recalibrer le rendement par la méthode indirecte qui est quasiment systématiquement supérieur de 1 à 2 points au rendement par la méthode directe. ${Q}_{cd} = \frac{{P}_{cd}}{100} * {Q}_{c} \left[kW\right]$
   et P_{cd}= coefficient forfaitaire de pertes proportionnelles en %

### Calcul de rendement simplifié

Dans une variante, si on ne dispose pas de l'information de débit des fumées, on peut tout de même calculer un rendement instantané de la chaudière en s'inspirant de la formule de Siegert pour les combustibles classiques. Il faut alors réaliser un bilan par unité de combustible et estimer les pertes par rayonnement. Par contre, on ne pourra en aucun cas obtenir les informations de puissance instantanée et charge de la chaudière bois.

Dans cette configuration, l'information de puissance instantanée de la chaudière Q_{c}, même si on en dispose, ne peut être utilisée dans les calculs, car pour cela il faudrait pouvoir la rapporter à une unité de combustible et donc disposer du débit du combustible.

Dans ce paragraphe, nous les effectuerons avec des coefficients de puissance par unité de combustible : ${G}_{b} + {G}_{a} = {G}_{c} + {G}_{f} + {G}_{r} + {G}_{cd} + {G}_{i} \left[kJ/kg bois brut\right]$
les indices ayant les mêmes significations que pour l'équation [45c]. De la même manière que ci-dessus nous introduirons G_{cd} qui permettra de prendre en compte des pertes forfaitaires non intégrées par ailleurs.

### Energie apportée par l'air Gₐ

### Calcul de Gₐ :

${G}_{a} = \frac{{VAH}_{r}}{{M}_{b}} . \frac{1}{{V}_{m}} {\int }_{298}^{Ta} {Cp}_{a} ⁢ dT \left[kJ/kg bois brut\right]$
avec :
*VAHᵣ* : volume comburivore réel en air humide [Nm3/mol bois sec sans cendres] (formule [13])
*M_{b} :* Masse de bois brut pour une mole de bois sec sans cendres [kg/mol bois sec sans cendres] (formule [22])
*Tₐ :* température de l'air comburant [K]
*298 K :* référence de température pour le calcul du PCI $\frac{1}{{V}_{m}} {\int }_{298}^{Ta} {Cp}_{a} ⁢ dT = \frac{1}{{V}_{m}} {\sum }_{i} {x}_{i} {\int }_{298}^{Ta} {Cp}_{i} ⁢ dT \left[kJ/{Nm}^{3}\right]$
   et avec : ${\int }_{298}^{Ta} {Cp}_{i} ⁢ dT en provenance de lʹéquation \left[45⁢b\right]$

### Energie apportée par le combustible G_{b}

### Calcul de G_{b} :

${G}_{b} = PCI \left[kJ/kg bois\right]$
Avec PCI donné formule [27]

### Pertes par les fumées

L'énergie perdue par les fumées G_{f} est calculée par la formule : ${G}_{f} = \frac{\left({VFH}_{r}+{V}_{{H}_{2} ⁢ O}\right)}{{M}_{b}} . \frac{1}{{V}_{m}} ⋅ {\int }_{298}^{Tf} {Cp}_{fumées} ⁢ dT \left[kJ/kg bois\right]$
avec
*VFHᵣ* : Volume fumigène humide réel [Nm³/mol bois sec sans cendres] (formule [14])
v_{H2O} : Volume d'eau évaporé par mol de bois [Nm³/mol bois sec sans cendres] (formule [19])
*T_{f}*: température des fumées [K]
*298 K :* référence de température pour le calcul du PCI $\frac{1}{{V}_{m}} {\int }_{298}^{Tf} {Cp}_{f} ⁢ dT$ en provenance des formules [38a] et [38b]

### Pertes par rayonnement

Les pertes par rayonnement d'une chaudière (formule [39]), sont un terme constant qui ne dépend quasiment pas de l'allure de la chaudière (la température des parois sera à peu près la même à toutes les allures).

Il est donc difficile de les intégrer dans un bilan par unité de combustible où elles auront un impact par exemple deux fois moindre à pleine charge qu'à demi-charge. On peut par exemple estimer la charge de la chaudière et évaluer les pertes par rayonnement par unité de combustible au prorata. ${G}_{r} = \frac{{Q}_{r}}{{D}_{nomcomb} * Charge} \left[kJ/kg bois\right]$

Avec D_{nomcomb}, débit nominal massique de la chaudière en combustible (donnée constructeur) (kg/h)
Charge : charge de la chaudière à l'instant t (%)
Qᵣ : pertes par rayonnement forfaitaires (donnée constructeur) (kW)

### Pertes forfaitaires G_{cd}

${G}_{cd} = \frac{{P}_{cd}}{100} * {G}_{c} \left[kW/kg bois\right]$
P_{cd}= coefficient forfaitaire de pertes proportionnelles en %

### Energie chaudière G_{c}

L'énergie fournie G_{c} sera évaluée par différence : ${G}_{c} = {G}_{b} + {G}_{a} - {G}_{f} - {G}_{r} - {G}_{cd} - {G}_{i} \left[kJ/kg bois\right]$ ${G}_{c} = \frac{{G}_{b} + {G}_{a} - {G}_{f} - {G}_{r} - {G}_{i}}{\left(1+{P}_{cd}\right)} \left[kJ/kg bois\right]$
en négligeant Gᵢ

### Rendement η

Le rendement s'exprimera toujours : $η = \frac{{G}_{c}}{{G}_{b}}$

L'invention permet de mettre en oeuvre un procédé de régulation de fonctionnement de la chaudière, en agissant d'une part sur le débit de combustible, et d'autre part sur la quantité d'air comburant insufflé par les moyens de soufflage 3, ainsi que sa répartition en air primaire sous le lit de combustion, en air secondaire et éventuellement tertiaire dans les flammes ou les fumées. Le réglage de ces paramètres est effectué en prenant en compte notamment une indication de la puissance, par exemple la puissance fournie par la matière organique ou la puissance de la chaudière, et l'humidité des fumées.

## Revendications

1. Procédé de détermination d'un taux d'humidité d'une matière organique utilisée comme combustible dans une chaudière comportant un conduit de fumées (4) pour évacuer des fumées de combustion de la matière organique dans la chaudière, **caractérisé en ce qu'**on réalise un prélèvement de fumées dans le conduit de fumées (4), on détermine le taux d'humidité dans les fumées prélevées et on détermine le taux d'humidité de la matière organique en fonction du taux d'humidité des fumées.

2. Procédé selon la revendication 1, selon lequel on réalise une première mesure d'un taux d'oxygène (B) dans les fumées prélevées et une deuxième mesure du taux d'oxygène (A2) dans les fumées prélevées au même endroit et après un assèchement desdites fumées, le taux d'humidité dans les fumées étant déterminé en fonction des mesures des taux d'oxygène (A2, B).

3. Procédé selon la revendication 2, selon lequel les fumées prélevées passent par une première sonde (51) à oxygène pour réaliser la première mesure de taux d'oxygène (B), par un assécheur (53) puis par une deuxième sonde (52) pour réaliser la deuxième mesure de taux d'oxygène (A2).

4. Procédé selon la revendication 3, selon lequel on aspire les fumées par une pompe (54) placée en aval de la deuxième sonde (52) à oxygène.

5. Procédé selon la revendication 3 ou 4, selon lequel l'assécheur (53) fonctionne par un refroidissement à température inférieure à 5°C.

6. Procédé selon l'une des revendications précédentes, selon lequel on déduit de l'humidité de la matière organique un pouvoir calorifique estimé de ladite matière organique.

7. Procédé selon la revendication 6, selon lequel on mesure en outre un débit massique ou volumique des fumées et un taux d'oxygène dans les fumées et on en déduit, en combinaison avec le pouvoir calorifique estimé, un débit massique de matière organique et une puissance fournie par la matière organique.

8. Procédé selon la revendication 7, selon lequel on mesure la puissance délivrée par la chaudière.

9. Procédé selon la revendication 7, dans lequel on déduit de la mesure de débit et d'une mesure de la température des fumées une puissance de perte par les fumées, on estime par ailleurs les pertes thermiques de la chaudière et on en déduit une puissance délivrée par la chaudière en déduisant de la puissance fournie par la matière organique les puissances de perte.

10. Procédé selon la revendication 9, selon lequel on retire de la puissance de la chaudière en outre une perte forfaitaire.

11. Procédé selon la revendication 10, selon lequel la perte forfaitaire est proportionnelle à la puissance délivrée par la chaudière.

12. Procédé selon l'une des revendications 8 ou 9, selon lequel on déduit de la puissance de la chaudière et de la puissance fournie par la matière organique, un rendement instantané de la chaudière.

13. Procédé selon la revendication 12, dans lequel le débit de combustible est ajusté en temps réel en fonction d'au moins un des paramètres suivants : rendement instantané de la chaudière, puissance de la chaudière, puissance fournie par la matière organique.

14. Procédé selon l'une des revendications précédentes, selon lequel la matière organique est du bois.

15. Système de détermination d'un taux d'humidité d'une matière organique utilisée comme combustible dans une chaudière, la chaudière comportant un conduit de fumées (4) pour évacuer des fumées de combustion de la matière organique, le système étant **caractérisé en ce qu'**il comporte des moyens de prélèvement (5) de fumées dans le conduit de fumées (4), et des moyens de calcul 6 aptes à déterminer un taux d'humidité de la matière organique en fonction d'un taux d'humidité des fumées mesuré ou déduit de mesures dans les fumées prélevées, les moyens de calcul 6 mettant en oeuvre le procédé selon l'une des revendications 1 à 14.
